# EUROPEAN PATENT APPLICATION

(11) **EP 2 815 742 A1**
(43) Date of publication of application: **24.12.2014**
(21) Application number: 13749007.4
(22) Date of filing: 01.02.2013
(51) Int. Cl.: A61K 9/127, A61K 36/896, A61P 1/04, A61P 1/00

(54) **IMPROVED DOSAGE FORM CONTAINING EXTRACT FROM BARK OF LIRIODENDRON TULIPIFERA AS ACTIVE INGREDIENT**

(30) Priority: 16.02.2012 KR 20120015770
(71) Applicant: Cho Dang Pharm. Co., Ltd., Seoul 152-050 (KR)
(72) Inventor: KIM, Nak Doo, Seoul 137-764 (KR)
(74) Representative: Harding, Charles Thomas
(86) International application number: PCT/KR2013/000821
(87) International publication number: WO 2013/122345

(57) **Abstract**

The present invention relates to an improved dosage form containing, as active ingredients, epitulipinolide and costunolide extracted from the bark of Liriodendron tulipifera and, more specifically, to a dosage form for treating the gastrointestinal tract, the dosage form containing, as active ingredients, epitulipinolide and costunolide, which are the active ingredients of the extract from the bark of Liriodendron tulipifera.

## Description

This is a national phase application of PCT/KR2013/000821 filed on February 1, 2013, claiming priority to Korean Patent Application No.10-2012-0015770 filed on February 16, 2012, the contents of which are incorporated herein by references.

### Technical Field

The present invention relates to a pharmaceutical formulation containing epitulipinolide and costunolide extracted from bark of Liriodendron tulipifera as active ingredient. More particularly, the present invention relates to a pharmaceutical formulation for treating the gastrointestinal disease containing epitulipinolide and costunolide extracted from bark of Liriodendron tulipifera as active ingredient.

### Description of Prior Art

It has been reported that the gastritis and/or stomach ulcer is the most common disease, because about 10% of Korean populations have suffered from gastritis and/or stomach ulcer more than once in a whole life. The causes of gastritis and/or stomach ulcer have been known by the digestion of stomach wall containing mucosa, sub-mucosa, muscularis externa and serosa by gastric acid. Gastritis has been referred if only gastric mucosa has been damaged, while stomach ulcer has been referred if gastric sub-mucosa and muscularis externa have been damaged. Further, the unbalance between offensive factor and defensive factor may cause the generation of gastritis and/or stomach ulcer, for example, the increase of offensive factor or the decrease of defensive factor.

The increase of secretion of gastric acid and/or pepsin can be an example of increase of offensive factor, while the defect of gastric mucosa, the decrease of mucus secretion, the decrease of bicarbonate ion secretion and/or the decrease of prostaglandin production can be an example of decrease of defensive factor. On the other hand, it has been known that the infection of Helicobacter pylori can cause the gastritis and/or stomach ulcer.

Gastric acid hyper-secretion has been regarded as the main cause of gastritis and/or stomach ulcer. The gastric acid, which is hydrochloric acid secreted from stomach wall cells has a role for digesting proteins by activating pepsin as well as damaging the stomach wall. The therapeutic agent for treating gastritis and/or stomach ulcer can be classified into the drug inhibiting the offensive factor, for example, gastric acid, pepsin, smoke, oxygen free radical and/or alcohol, and the drug enhancing the defensive factor.

As an offensive factor inhibitor, antacid and/or gastric acid secretion inhibitor can be exemplified. Further, as a gastric acid secretion inhibitor, the antagonist of H2 receptor, for example, cimetidine, ranitidine and/or famotidine etc.; the proton pump inhibitor (PPI), for example, omeprazole and/or lansoprazole can be exemplified. Especially, almost 100% of duodenal ulcer and more than 90% of stomach ulcer can be initially recovered by administration of PPI drug. However, in spite of improving initial recovery rate of duodenal ulcer and/or stomach ulcer, the recurrence rate has not been reduced.

On the other hand, a defensive factor enhancer can induce the covering of the ulcerative lesion, the synthesis and secretion of mucus, the increase of blood flow in gastric mucosa, the increase of endogenous prostaglandin and/or the increase of tissue regeneration. Further, it has been known that a defensive factor enhancer has been useful for preventing the recurrence of duodenal ulcer and/or stomach ulcer. Of course, the sterile therapy of Helicobacter pylori also can be considered as important treating method against ulcer.

However, the causes of gastritis and/or stomach ulcer have not been clearly disclosed since complex causes result in gastritis and/or stomach ulcer. Therefore, the absolute treating method has not been developed.

On the other hand, Liriodendron tulipifera L. is a kind of deciduous broad-leaved arboreal belonged to Magnoliaceae family. It has been used as a raw material of pulp. In the bark, leave and/or wood of Liriodendron tulipifera, alkaloid, sesquiterpene and/or lignan has been known to be included.

It has been known that aporphine alkaloid having anti-bacterial activity has been contained in the bark of Liriodendron tulipifera (Hufford C. D. and Funderburk M. J., J. Pharm. Sci. 63: pp.1338-1339, 1974; Hufford C. D. et al., J. Pharm. Sci. 64: pp.789-792, 1975). Further, the bark extract containing this alkaloid has been used for treating malaria since Unites States War of Independence. On the other hand, glaucine alkaloid has been used for antitussive agent in Soviet Union.

According to the research in 1975, the alkaloid fraction extracted from the bark of Liriodendron tulipifera has been reported as having anti-bacterial activity. Further, it has been reported that N-methyllaurotetanine, lirioferine and/or liriotulipiferine has an anti-fungal activity (Huang Hsu, C-Y, 1976. MS thesis, North Carlolina State University, Raleigh). Further, it has been also reported that dehydroglaucine and/or liriodenine has cytotoxicity and anti-bacterial activity (Warthen D, Gooden E L and Jacobson M., J. Pharm. Sci., 58: pp.637-638(1969); Hufford C D, Funderburk M J, Morgan J M. et al., J. Pharm. Sci., 64: pp.789-792(1975); Chen C R, Bed J L, Doskotch R W et al., Lloydia 37: pp.493-500(1974)).

On the other hand, it has been also disclosed that sesquiterpene lactone extracted from bark of Liriodendron tulipifera has an anti-tumor activity. Further, costunolide, tulipinolide, epi-tulipinolide, epi-tulipdienolide and/or gamma-liriodenolide has been also disclosed as an effective ingredient of the bark of Liriodendron tulipifera (Doskotch R. W and EL-Feraly F. S., J Pharm. Sci. 58: pp.877-880, 1969; Doskotch R. W and E LI-Feraly F. S., J. Org. Chem. 35: pp.1928-1936, 1970; Moon M. K. et al., Arch. Pharm .Res. 30: pp.299-302, 2007).

Further, in the leaf of Liriodendron tulipifera , lipiferolide, epitulipinolide diepoxide and/or peroxyferolide has been also disclosed as an effective ingredient (Doskoch R W, EL-Feraly F S, Fairchild E H, J. Chem. Soc (Chem. Comm) pp.402-403, 1976; Doskotch R W, EL-Feraly F S, Fairchild E H, J. Org. Chem. 42: pp.3614-3618, 1977). On the other hand, in the bark of Liriodendron tulipifera , lignan derivatives, pinoresinol, (+)-syringaresinol and/or liriodendrin in the form of monoglycoside or diglycoside has been also disclosed as an effective ingredient (Dickey E E., J. Org. Chem. 23: pp.179-184, 1958; Fujimoto H and Higuchi T., J. Jap. Wood Res. Soc, 23: pp.405-410, 1977). Further, it has been reported that liriodendrin causes the bone growth in animal (Korean patent registration No.10-597,563).

The inventor of present application has found that extract from the bark of Liriodendron tulipifera containing epitulipinolide and costunolide as active ingredients can be applied for treating gastritis and/or stomach ulcer. Further, various kinds of small amount materials have been isolated in the extract composition from the bark of Liriodendron tulipifera.

Therefore, the inventor of present application has prepared a pharmaceutical formulation containing epitulipinolide and costunolide as active ingredients from the bark of Liriodendron tulipifera for treating gastritis and/or stomach ulcer. Finally, the present invention has been completed by preparing a pharmaceutical formulation containing epitulipinolide and costunolide as active ingredients to be easily solubilized for improving dissolution rate.

### Problem to be solved

The problem to be solved is to develop a pharmaceutical formulation containing epitulipinolide and costunolide as active ingredients from the bark of Liriodendron tulipifera for treating gastritis and/or stomach ulcer. Accordingly, the development of pharmaceutical formulation containing epitulipinolide and costunolide as active ingredients from the bark of Liriodendron tulipifera has been carried out to be easily solubilized for improving dissolution rate.

### Means for solving the problem

The object of present application is to provide a pharmaceutical formulation for preventing and treating gastrointestinal disease comprising the extract from the bark of Liriodendron tulipifera / surfactant / co-solvent in the weight ratio of 1 / 0.1∼50 / 0.1∼50, wherein said extract from the bark of Liriodendron tulipifera comprises epitulipinolide and costunolide as active ingredients.

Further, said surfactant can be at least one selected from the group consisting of polyoxyethylene sorbitan fatty acid ester, sorbitan fatty acid ester, polyoxyethylene fatty acid ester, polyoxyethylene polyoxypropylene block copolymer, polyoxyethylene polyoxypropylene copolymer, the reaction product between natural or hydrogenated vegetable oil and ethylene glycol, dioctyl sodium sulfosuccinate, sodium lauryl sulfonate, phospholipids, propylene glycol mono-fatty acid ester, propylene glycol di-fatty acid ester, trans-esterified reaction product between natural vegetable oil triglyceride and polyalkylene polyol, mono-di glyceride, mono/di glyceride, sterol and derivative thereof.

Further, said co-solvent can be at least one selected from the group consisting of propylene carbonate, propylene glycol, ethanol, diethylene glycol monoethyl ether, glycofurol, polyethylene glycol, dimethyl isosorbide, and N-methyl pyrrolidone.

On the other hand, the weight ratio of the extract from the bark of Liriodendron tulipifera / surfactant / co-solvent is 1 / 0.5∼30 / 0.5- 30.

### Advantageous effect

The advantageous effect of present application is to provide a pharmaceutical formulation containing epitulipinolide and costunolide as active ingredients from the bark of Liriodendron tulipifera for treating gastritis and/or stomach ulcer. Further, the extract from the bark of Liriodendron tulipifera to be easily solubilized for improving dissolution rate has been provided.

On the other hand, the extract containing epitulipinolide and costunolide as active ingredients has an efficacy for preventing or treating gastrointestinal disease. Especially, the extract has an excellent efficacy for treating gastritis and/or stomach ulcer. Further, oral formulation of this extract of present application can accomplish the outstanding increase of bioavailability by increasing solubility and dissolution rate of insoluble components.

### Brief description of drawings

Fig. 1 shows the dissolution rate between soft capsule formulation (test formulation) and tablet formulation (comparison formulation) in pH=1.2 buffer solution according to Example 2 of present application. Each formulation contains 30 mg of extract from bark of Liriodendron tulipifera.
Fig. 2 shows the dissolution rate between soft capsule formulation (test formulation) and tablet formulation (comparison formulation) in pH=6.8 buffer solution according to Example 2 of present application. Each formulation contains 30 mg of extract from bark of Liriodendron tulipifera.

### Preferred embodiment of invention

The present invention is to provide a pharmaceutical formulation containing the extract from bark of Liriodendron tulipifera. The extract from the bark of Liriodendron tulipifera has been prepared using the extraction solvent selected from ethyl acetate, dichloromethane, alcohol, alcohol aqueous solution and/or mixture of them. Further, epitulipinolide and costunolide have been included in this extract. Of course, epitulipinolide and costunolide have been used as component indicator of the extract from the bark of Liriodendron tulipifera.

Of course, the extract from the bark of Liriodendron tulipifera has an excellent efficacy for treating gastrointestinal disease. For measuring the efficacy for treating gastritis and/or stomach ulcer, various kinds of in vivo animal test models have been employed. For example, water-immersion-restraint stress model, HCl-EtOH inducing gastritis model and/or indomethacin inducing stomach ulcer model has been employed. As a result of the test, the extracted material from the bark of Liriodendron tulipifera shows an excellent efficacy to the gastritis and/or stomach ulcer by promoting the prostaglandin biosynthesis.

On the other hand, the present invention provide a pharmaceutical composition from the bark of Liriodendron tulipifera comprising epitulipinolide and costunolide as active ingredients for preventing or treating gastrointestinal disease. Further, said pharmaceutical composition further comprises epitulipdienolide, ridentin and/or deacetyllipiferolide as another active ingredient.

The pharmaceutical composition can be administered in various routes, for example, oral, intravenous and/or intramuscular. The dose of pharmaceutical composition is 10∼500mg per one day.

Preferred formulation of pharmaceutical composition is an oral preparation which is at least one formulation selected from of tablet, pill, powder, hard capsule, gelatin banding hard capsule, soft capsule, chewing tablet in the form of caramel or jelly and/or oral solution. Further, soft capsule or oral solution is the most desirous.

The pharmaceutical composition containing the extract from the bark of Liriodendron tulipifera can additionally comprise surfactant and co-solvent. The weight ratio of the extract from the bark of Liriodendron tulipifera / surfactant / co-solvent is 1 / 0.1∼50 / 0.1- 50, preferably 1 / 0.5∼30 / 0.5- 30. Further, the oral formulation can be prepared in a conventional method, such as, mixing the extract, surfactant and co-solvent; heating and solubilizing the mixture.

The surfactant can make the automatic formation of micelle to the extract from the bark of Liriodendron tulipifera when the water insoluble type of extract from the bark of Liriodendron tulipifera is exposed into aqueous solution or body fluid. Therefore, both solubility and dissolution rate of insoluble type of extract can be enhanced. Of course, the physical and chemical stability of insoluble type of extract can be enhanced. Further, only minimum amount of surfactant can be included for the convenience of administration.

The kind of surfactant can include non-ion, cation and/or anion surfactant. Specifically, said surfactant can be at least one selected from the group consisting of sorbitan fatty acid ester (Span), polyoxyethylene sorbitan fatty acid ester (Polysorbate or Tween), polyoxyethylene fatty acid ester, Myrij, polyoxyethylene polyoxypropylene block copolymer, polyoxyethylene polyoxypropylene copolymer (poloxamer), dioctyl sodium sulfosuccinate, sodium lauryl sulfonate, phospholipids, propylene glycol mono-fatty acid ester, propylene glycol di-fatty acid ester, Miglyol 840, the reaction product between natural or hydrogenated vegetable oil and ethylene glycol(Cremophor), trans-esterified reaction product between natural vegetable oil triglyceride and polyalkylene polyol (Labrafil M), mono-di glyceride, mono/di glyceride (Imbitol), sterol and derivative thereof. Preferably, said surfactant can be selected from Cremophor, polyoxyethylene sorbitan fatty acid ester or polyoxyethylene polyoxypropylene block copolymer.

Further, said co-solvent can be at least one selected from the group consisting of propylene glycol, ethanol, diethylene glycol monoethyl ether, glycofurol, polyethylene glycol, dimethyl isosorbide, propylene carbonate and N-methyl pyrrolidone. Preferably, said co-solvent can be selected from propylene glycol, polyethylene glycol and diethylene glycol monoethyl ether.

Further, said pharmaceutical formulation can include pharmaceutically acceptable carriers. For example, the additive, such as, acid, fatty acid and/or fatty alcohol can be included to improve the solubility of extract from bark of Liriodendron tulipifera as well as to improve the dissolution rate of extract from bark of Liriodendron tulipifera by increasing its dispersion. Further, the sugar component, such as, white sugar, maltose, purified white sugar, starch syrup can be included. Of course, any inorganic additive, such as, magnesium stearate, talc as well as diluent, such as, non-crystalline cellulose, calcium hydrogen phosphate, starch and/or mannitol can be included. Further, anti-oxidant, flavoring agent, preservative, flavor, sweetener, pigment, pH adjusting agent and/or viscosity adjusting agent can be included. The amount of this additive has been conventionally known.

As the acid, the fatty acid or the fatty alcohol in said pharmaceutical formulation, citric acid, oleic acid, stearyl alcohol, myristic acid, linoleic acid, lauric acid, capric acid, caprylic acid and/or caproic acid can be used, but not be limited thereto.

As the antioxidant in said pharmaceutical formulation, butylated hydroxy toluene, sodium bisulfite, α-tocopherol, vitamin C, β-carotene, tocopherol acetate, fumarate acid, nalic acid, butylated hydroxyanisole, propyl gallate and/or sodium ascorbate can be used, but not be limited thereto.

As the flavoring agent in said pharmaceutical formulation, mixed fruit flavor, apple flavor, strawberry flavor, cherry flavor, mint, vanilla flavor, yogurt flavor, or drink flavor can be used, but not be limited thereto.

As the preservative in said pharmaceutical formulation, benzoic acid, sodium benzoate, ethylparaben, methylparaben or propylparaben can be used, but not be limited thereto.

As the flavor in said pharmaceutical formulation, menthol, peppermint oil, orange oil, clove oil, cinnamon oil, strawberry essence and/or other conventional plant extract or fruit aroma can be used, but not be limited thereto.

As the sweetener in said pharmaceutical formulation, refined sugar, glucose, fructose, aspartame, stevioside, sorbitol, mannitol, oligosaccharide and/or starch syrup can be used, but not be limited thereto.

As the pigment in said pharmaceutical formulation, Green No. 3, Red No. 2, Red No. 3, Blue No. 1, Blue No. 2, Yellow No. 4, Yellow No. 5, soluble mannitol, caramel, titanium oxide or ferric oxide can be used, but not be limited thereto.

As the pH adjusting agent in said pharmaceutical formulation, sodium carbonate, sodium hydroxide, potassium hydroxide, triethanolamine or monoethanolamine can be used, but not be limited thereto.

As the viscosity adjusting agent in said pharmaceutical formulation, hydroxypropyl cellulose (HPC), hydroxypropylmethylcellulose (HPMC), hydroxyethyl cellulose, ethyl cellulose, methyl cellulose, carboxymethyl cellulose, acacia, bentonite, alginic acid, propylene glycol alginate, polyvinylpyrrolidone, polyvinyl alcohol, carbopol, polycarbopil, tragacanth or xanthan can be used, but not be limited thereto.

The present invention can be explained more concretely by following Preparation Examples and Examples. However, the scope of present invention cannot be limited by following Examples.

### (Preparation Example 1) Preparation of isolated and purified extract from bark of Liriodendron tulipifera

200g of dried and chopped bark of Liriodendron tulipifera has been extracted using 800ml of ethyl acetate at 50 °C for 24 hours. Obtained extracted solution has been concentrated and dried at reduced pressure. Finally, 6.83 g of extract has been obtained. The amount of epitulipinolide has been 3.96 wt% and the amount of costunolide has been 0.18 wt%.

1g of extract obtained in above step has been dissolved with 50ml of 70% ethanol. After adding 50ml of hexane, the mixture has been agitated and fractioned. Obtained 70% ethanol fraction has been isolated and dried. Finally, 0.483g of purified extract has been obtained. The amount of epitulipinolide has been 9.72 wt% and the amount of costunolide has been 0.28 wt%.

### (Preparation Example 2) Preparation of isolated and purified extract from bark of Liriodendron tulipifera

200g of dried and chopped bark of Liriodendron tulipifera has been extracted using 800ml of ethyl acetate at 50°C for 48 hours. Obtained extracted solution has been concentrated and dried at reduced pressure. Finally, 7.67g of extract has been obtained. The amount of epitulipinolide has been 3.94 wt% and the amount of costunolide has been 0.14 wt%.

1g of extract obtained in above step has been dissolved with 50ml of 70% ethanol. After adding 50ml of hexane, the mixture has been agitated and fractioned. Obtained 70% ethanol fraction has been isolated and dried. Finally, 0.571g of purified extract has been obtained. The amount of epitulipinolide has been 10.94 wt% and the amount of costunolide has been 0.30 wt%.

### (Preparation Example 3) Preparation of isolated and purified extract from bark of Liriodendron tulipifera

200g of dried and chopped bark of Liriodendron tulipifera has been extracted using 800ml of dichloromethane at 36°C for 72 hours. Obtained extracted solution has been concentrated and dried at reduced pressure. Finally, 4.95g of extract has been obtained. The amount of epitulipinolide has been 5.10 wt% and the amount of costunolide has been 0.35 wt%.

1g of extract obtained in above step has been dissolved with 50ml of 70% ethanol. After adding 50ml of hexane, the mixture has been agitated and fractioned. After obtaining ethanol fraction, 40ml of water and 90ml of dichloromethane have been added and agitated. Obtained dichloromethane fraction has been isolated and dried. Finally, 0.071g of purified extract has been obtained. The amount of epitulipinolide has been 23.43 wt% and the amount of costunolide has been 0.79 wt%.

### (Preparation Example 4) Preparation of isolated and purified extract from bark of Liriodendron tulipifera

200g of dried and chopped bark of Liriodendron tulipifera has been extracted using 800ml of dichloromethane at 36°C for 24 hours. Obtained extracted solution has been concentrated and dried at reduced pressure. Finally, 9.12g of extract has been obtained. The amount of epitulipinolide has been 9.11wt% and the amount of costunolide has been 0.29 wt%.

1g of extract obtained in above step has been dissolved with 50ml of 70% ethanol. After adding 50ml of hexane, the mixture has been agitated and fractioned. After obtaining ethanol fraction, 40ml of water and 90ml of dichloromethane have been added and agitated. Obtained dichloromethane fraction has been isolated and dried. Finally, 0.069g of purified extract has been obtained. The amount of epitulipinolide has been 21.19 wt% and the amount of costunolide has been 0.94 wt%.

In the extracted materials of Preparation Examples, epitulipdienolide (Mw=290), ridentin (Mw=264), deacetyllipiferolide (Mw=264) can be additionally included.

### (Preparation Example 5) Oral formulation containing the extract from bark of Liriodendron tulipifera

30mg of the extract from bark of Liriodendron tulipifera obtained in Preparation Example 1, 80mg of polyoxyethylene sorbitan fatty acid ester and 80mg of Cremophor EL have been mixed. After adding 95mg of diethylene glycol monoethyl ether, the mixture has been heated at 80°C until the mixture has become transparent. Finally, the oral formulation has been prepared by filling the obtained mixture into the soft capsule.

### (Preparation Example 6) Oral formulation containing the extract from bark of Liriodendron tulipifera

30mg of the extract from bark of Liriodendron tulipifera obtained in Preparation Example 2 and 40mg of polyoxyethylene sorbitan fatty acid ester have been mixed. After adding 100mg of Cremophor RH40 and 100mg of diethylene glycol monoethyl ether, the mixture has been heated at 80°C until the mixture has become transparent. Finally, the oral formulation has been prepared by filling the obtained mixture into the soft capsule.

### (Preparation Example 7) Oral formulation containing the extract from bark of Liriodendron tulipifera

30mg of the extract from bark of Liriodendron tulipifera obtained in Preparation Example 3, 40mg of polyoxyethylene sorbitan fatty acid ester and 10mg of polyoxyethylene polyoxypropylene block copolymer have been mixed. After adding 100mg of Cremophor RH40 and 100mg of diethylene glycol monoethyl ether, the mixture has been heated at 80°C until the mixture has become transparent. Finally, the oral formulation has been prepared by filling the obtained mixture into the soft capsule.

### (Preparation Example 8) Oral formulation containing the extract from bark of Liriodendron tulipifera

30mg of the extract from bark of Liriodendron tulipifera obtained in Preparation Example 4, 100mg of polyoxyethylene sorbitan fatty acid ester and 100mg of polyoxyethylene polyoxypropylene block copolymer have been mixed. After adding 150mg of polyethylene glycol 400 and 600mg of propylene glycol, the mixture has been heated at 80°C until the mixture has become transparent. Further, 10,000mg of white sugar, 30mg of citric acid and 5mg of mixed fruit flavor have been added. Subsequently, an amount of purified water has been added and sterilized. Finally, the oral formulation has been prepared by filling the obtained solution into the bottle.

### (Preparation Example 9) Oral formulation containing the extract from bark of Liriodendron tulipifera

30mg of the extract from bark of Liriodendron tulipifera obtained in Preparation Example 1, 350mg of polyoxyethylene sorbitan fatty acid ester and 200mg of polyoxyethylene polyoxypropylene block copolymer have been mixed. After adding 380mg of polyethylene glycol 400 and 20mg of propylene glycol, the mixture has been heated at 80°C until the mixture has become transparent. Further, 1,000mg of malto ion syrup, 1,000mg of white sugar, 100mg of gelatin, 500mg of sugar, 100mg of syrup, 5mg of strawberry essence and 5mg of citric acid have been added. Finally, the oral formulation has been prepared by formulating caramel type chewing tablet.

### (Comparative Preparation Example 1) Oral formulation containing the extract from bark of Liriodendron tulipifera

30mg of the extract from bark of Liriodendron tulipifera obtained in Preparation Example 1, 30mg of Cremophor EL and 100mg of colloidal silicon dioxide (Aerosil 200) have been mixed and dried. After adding 70mg of Povidone, 90mg of none crystalline cellulose and 2mg of magnesium stearate, the mixture has been compressed. Finally, oral tablet has been prepared.

### (Comparative Preparation Example 2) Oral formulation containing the extract from bark of Liriodendron tulipifera

30mg of the extract from bark of Liriodendron tulipifera obtained in Preparation Example 1, 30mg of Cremophor EL and 70mg of colloidal silicon dioxide (Aerosil 200) have been mixed and dried. After adding 30mg of Povidone, 100mg of none crystalline cellulose and 2mg of magnesium stearate, the mixture has been compressed. Finally, oral tablet has been prepared.

### (Comparative Preparation Example 3) Oral formulation containing the extract from bark of Liriodendron tulipifera

30mg of the extract from bark of Liriodendron tulipifera obtained in Preparation Example 1, 30mg of Cremophor EL and 70mg of colloidal silicon dioxide (Aerosil 200) have been mixed and dried. After adding 30mg of Povidone, 100mg of none crystalline cellulose and 2mg of magnesium stearate, the mixture has been compressed. Finally, oral tablet has been prepared.

### (Example 1) Solubility comparison test

Oral formulation before filling the soft capsule prepared from Preparation Example 5 and oral formulation before compressing the tablet prepared from Comparative Preparation Example 1 have been employed as comparison test material for measuring the solubility.

The solubility has been measured in stomach pH condition (pH=1.2) as well as intestinal pH condition (pH=6.8). Experimental method can be explained as follows.

2 test materials have been employed in the same amount of oral formulation containing 60mg of the extract from bark of Liriodendron tulipifera prepared from Preparation Example 5 as well as Comparative Preparation Example 1. The pH 1.2 buffer solution has been prepared by mixing 2.0g of sodium chloride, 7.0ml of hydrochloric acid and water to make total volume into 1L according to the Korean Pharmacopoeia 8th edition, while the pH 6.8 buffer solution has been prepared by mixing 250ml of potassium dihydrogen phosphate solution 0.2mol/L, 118ml of sodium hydroxide solution 0.2mol/L and water to make total volume into 1 L according to the Korean Pharmacopoeia 8th edition. The test materials have been added into 150ml of pH 1.2 buffer solution and pH 6.8 buffer solution respectively. The mixture has been stirred for 1 hour at 37°C. The solubility has been measured using 270nm of UV after filtration of mixture. The results have been shown in Table 1.

**[Table 1] Solubility comparison in pH 1.2 and pH 6.8**

| Formulation | Solubility (µg/ml) | |
|---|---|---|
| | pH 1.2 | pH 6.8 |
| Material in Prep. Example 5 (test material) | 400 | 400 |
| Material in Com. Prep. Example 1 (comparison material) | 197 | 191 |
| Solubility comparison (Prep. Example 5 / Com. Prep. Example 1) | more than twice | more than twice |

As shown in Table 1, the solubility of oral formulation prepared in Preparation Example 5 (test material) has been shown 400µg/ml in pH 1.2 and 400µg/ml in pH 6.8 respectively, while the solubility of oral formulation prepared in Comparative Preparation Example 1 (comparison material) has been shown 197µg/ml in pH 1.2 and 191µg/ml in pH 6.8 respectively. From this solubility test, the solubility of test material in present invention has been shown more than twice higher than that of comparison material in pH 1.2 buffer solution as well as pH 6.8 buffer solution. Automatic micelle drug delivery system according to present invention can make such increase of solubility in the test material. Therefore, it has been proved that the oral formulation prepared according to the method of present invention can show high solubility in various kinds of pH conditions.

### (Example 2) Dissolution rate comparison test

Oral formulation before filling the soft capsule prepared from Preparation Example 5 and oral formulation before compressing the tablet prepared from Comparative Preparation Example 1 have been employed as comparison test material for measuring the dissolution rate. The dissolution rate has been measured in stomach pH condition (pH=1.2) as well as intestinal pH condition (pH=6.8). Experimental method can be explained as follows.

2 test materials have been employed in the same amount of oral formulation containing 30mg of the extract from bark of Liriodendron tulipifera prepared from Preparation Example 5 as well as Comparative Preparation Example 1. The pH 1.2 buffer solution and the pH 6.8 buffer solution have been prepared as the same manner of Example 1. The test materials have been added into 900ml of pH 1.2 buffer solution and pH 6.8 buffer solution respectively. The mixture has been stirred in 50rpm. According to Paddle method, the sample has been collected at 5 min., 10 min., 15 min., 30 min., 45 min., 60 min., 90 min. and 120 min. after to measure the amount of the extract from the bark of Liriodendron tulipifera. The dissolution rate has been calculated in the percent ratio from the measured amount of the extract from the bark of Liriodendron tulipifera, on condition that the amount of the extract from the bark of Liriodendron tulipifera in oral formulation is 100.

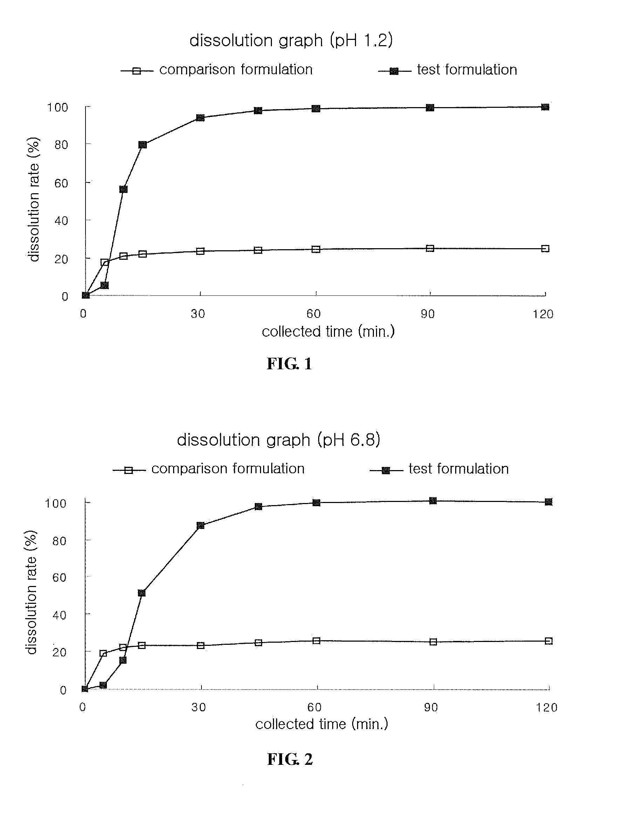

Fig. 1 shows the dissolution rate measured in pH=1.2 condition, while Fig. 2 shows the dissolution rate measured in pH=6.8 condition.

As shown in Fig. 1, the dissolution rate of oral formulation prepared in Preparation Example 5 (test material) has been shown more than 60% dissolution rate at 10 min. and more than 70% dissolution rate at 15-120 min. in pH 1.2 while the dissolution rate of oral formulation prepared in Comparative Preparation Example 1 (comparison material) has been shown very low. Automatic micelle drug delivery system according to present invention can make such increase of dissolution rate in the test material. Therefore, it has been proved that the oral formulation prepared according to the method of present invention can show high dissolution rate in pH=1.2 buffer solution.

As shown in Fig. 2, the dissolution rate of oral formulation prepared in Preparation Example 5 (test material) has been shown more than 60% dissolution rate at 15 min. and more than 70% dissolution rate at 15∼60 min. in pH 6.8 while the dissolution rate of oral formulation prepared in Comparative Preparation Example 1 (comparison material) has been shown very low. Automatic micelle drug delivery system according to present invention can make such increase of dissolution rate in the test material. Therefore, it has been proved that the oral formulation prepared according to the method of present invention can show high dissolution rate in pH=6.8 buffer solution.

As shown in Fig. 1 and Fig. 2, automatic micelle drug delivery system according to present invention can make such increase of dissolution rate in pH=1.2 buffer solution as well as pH=6.8 buffer solution. Therefore, it has been proved that the oral formulation prepared according to the method of present invention can show high dissolution rate in various kinds of pH buffer solution compared to conventional oral formulation prepared in Comparative Preparation Example.

## Claims

1. A pharmaceutical formulation for preventing and treating gastrointestinal disease comprising the extract from the bark of Liriodendron tulipifera / surfactant / co-solvent in the weight ratio of 1 / 0.1∼50 / 0.1- 50, wherein said extract from the bark of Liriodendron tulipifera comprises epitulipinolide and costunolide as active ingredients.

2. The pharmaceutical formulation according to claim 1, wherein said surfactant is at least one selected from the group consisting of polyoxyethylene sorbitan fatty acid ester, sorbitan fatty acid ester, polyoxyethylene fatty acid ester, polyoxyethylene polyoxypropylene block copolymer, polyoxyethylene polyoxypropylene copolymer, the reaction product between natural or hydrogenated vegetable oil and ethylene glycol, dioctyl sodium sulfosuccinate, sodium lauryl sulfonate, phospholipids, propylene glycol mono-fatty acid ester, propylene glycol di-fatty acid ester, trans-esterified reaction product between natural vegetable oil triglyceride and polyalkylene polyol, mono-di glyceride, mono/di glyceride, sterol and derivative thereof.

3. The pharmaceutical formulation according to claim 1, wherein said co-solvent is at least one selected from the group consisting of propylene carbonate, propylene glycol, ethanol, diethylene glycol monoethyl ether, glycofurol, polyethylene glycol, dimethyl isosorbide, and N-methyl pyrrolidone .

4. The pharmaceutical formulation according to claim 1, wherein the weight ratio of the extract from the bark of Liriodendron tulipifera / surfactant / co-solvent is 1 / 0.5∼30 0.5∼30.
